# FASCICULE DE BREVET EUROPEEN

(11) **EP 1 214 313 B1**
(45) Date de publication et mention de la délivrance du brevet: **30.06.2004**
(21) Numéro de dépôt: 00962583.1
(22) Date de dépôt: 08.09.2000
(51) Int. Cl.: C07D 401/04, C07D 401/14, A61K 31/4545, A61K 31/4523, A61P 1/00

(54) **HETEROARYLOXYPROPANOLAMINES COMME AGONISTES DU RECEPTEUR BETA3-ADRENERGIQUE**
HETEROARYLOXYPROPANOLAMINE ALS BETA3-ADRENERGISCHER REZEPTOR-AGONISTEN
HETEROARYLOXY PROPANOLAMINES, PREPARATION METHOD AND PHARMACEUTICAL COMPOSITIONS CONTAINING SAME

(30) Priorité: 08.09.1999 FR 9911204
(43) Date de publication de la demande: 19.06.2002
(73) Titulaire: SANOFI-SYNTHELABO, 75013 Paris (FR)
(72) Inventeur: CECCHI, Roberto, I-26900 Lodi (IT); OLIVA, Ambrogio, I-21047 Sarrono (IT)
(74) Mandataire: Varady, Peter
(86) Numéro de dépôt international: PCT/FR2000/002482
(87) Numéro de publication internationale: WO 2001/017989

(56) Documents cités:
- EP-A- 0 095 454
- DE-A- 3 524 955
- US-A- 5 627 196
- M HORI ET AL: "A soluble polymer approach to the fishing out principle: synthesis and purification of beta-amino alcohols" JOURNAL OF ORGANIC CHEMISTRY,US,AMERICAN CHEMICAL SOCIETY. EASTON, vol. 63, no. 3, 6 février 1998 (1998-02-06), pages 889-894-894, XP002110346 ISSN: 0022-3263 cité dans la demande

## Description

La présente invention concerne de nouvelles propanolamines, les compositions pharmaceutiques les contenant, un procédé pour leur préparation et des intermédiaires dans ce procédé.

Ces nouveaux composés ont montré une activité agoniste vis-à-vis du récepteur β₃ et peuvent donc être utilisés dans le traitement des pathologies qui bénéficient de l'activation de ce récepteur.

BE 902897 décrit des aryloxypropanolamines portant un groupe 4-pipéridininyl-1-substitué sur l'amine, ces composés ayant une activité β₁-bloquante et α-bloquante.

J. Org. Chem., 1988, 63 :889 :894 décrit d'autres aryloxypropanolamines portant un groupe 4-pipéridinyl-1-substitué sur l'amine.

Il a été maintenant trouvé que des hétéroaryloxypropanolamines portant un radical pipéridin-4-yle ou pipéridin-4-ylalkylène sur l'amine possèdent une activité agoniste vis-à-vis des récepteurs β₃-adrénergiques.

Ainsi la présente invention concerne, selon un de ses aspects, des propanolamines de formule (I) 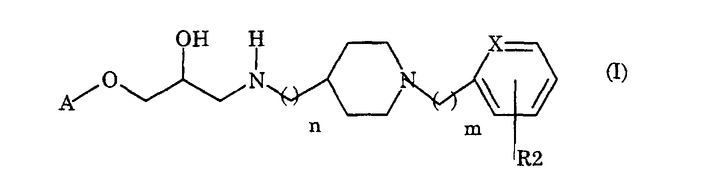
- où X: est N ou CH ;
- A: représente un groupe de formule (a) ou (b) 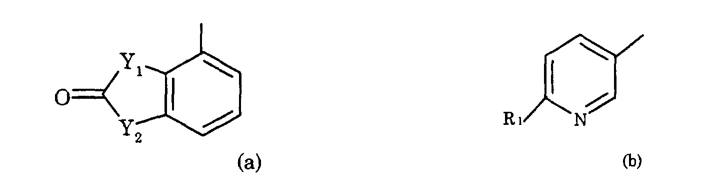
- R₁: représente un atome d'hydrogène, ou un groupe -NH₂, -NR₃R₄, -NR₃CO(C₁-C₄) Alk ou -NR₃SO₂(C₁-C₄)Alk;
- R₂: représente un atome d'hydrogène ou d'halogène, un groupe (C₁-C₄)Alk, (C₁-C₄)alcoxy, -COOH, -COO(C₁-C₄)Alk, -CN, -CONR₃R₄, -NO₂, -SO₂NR₃R₄, ou -NHSO₂(C₁-C₄)Alk;
- m et n: représentent chacun 0, 1 ou 2 ;
- R₃ et R₄: représentent chacun un atome d'hydrogène ou un groupe (C₁-C₄)Alk;
- Y₁ et Y₂: représentent chacun NH ou O ;
et leur sels ou solvates.

Dans la présente description le terme "(C₁-C₄)Alk" désigne un radical monovalent d'un hydrocarbure en C₁-C₄ saturé à chaîne droite ou ramifiée.

Les sels des composés de formule (I) selon la présente invention comprennent aussi bien les sels d'addition avec des acides mineraux ou organiques pharmaceutiquement acceptables tels que le chlorhydrate, le bromhydrate, le sulfate, l'hydrogénosulfate, le dihydrogénophoshate, le citrate, le maléate, le tartrate, le fumarate, le gluconate, le méthanesulfonate, le 2-naphtalènesulfonate, etc., que les sels d'addition qui permettent une séparation ou une cristallisation convenable des composés de formule (I), tels que le picrate, l'oxalate ou les sels d'addition avec des acides optiquement actifs, par exemple les acides camphosulfoniques et les acides mandéliques ou mandéliques substitués.

De plus, lorsque les composés de formule (I) possèdent un groupe carboxy libre les sels comprennent aussi les sels avec des bases minérales, de préférence les sels obtenus avec des bases de métaux alcalins tel que le sodium ou le potassium, ou avec des bases organiques.

Les stéréoisomères optiquement purs, ainsi que les mélanges d'isomères des composés de formule (I), font partie de la présente invention.

Des composés préférés de la présente invention comprennent les composés de formule (I) où X représente CH.

D'autres composés préférés de la présente invention sont ceux où X représente l'azote et le groupe R₂ est dans la position 5.

D'autres composés préférés sont ceux où le groupe (C₁-C₄)Alk est un groupe méthyle ou éthyle.

D'autres composés préférés sont ceux où R₂ est choisi parmi -COOH, -COO(C₁-C₄)Alk, -CN, -NO₂, -CONR₃R₄, -NHSO₂-(C₁-C₄)Alk, Cl.

D'autres composés préférés encore sont ceux ou n et m sont chacun zéro.

Particulièrement avantageux est le composé 3-[1-(5-éthoxycarbonylpyrid-2-yl)-4-pipéridinylamino]-1-[1,2-dihydro-2-oxo-benzimidazol-4-yloxy]-2-propanol, éventuellement salifié.

Un autre composé particulièrement avantageux est le 3-[1-(5-éthoxycarbonylpyrid-2-yl)-4-pipéridinylamino]-1-[2-amino-pyrid-5-yloxy]-2-propanol, éventuellement salifié.

Les composés de formule (I) sont préparés en traitant un époxyde de formule (II) : 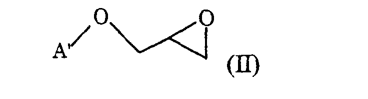 où A' représente le groupe (a) ou le groupe (b) dans lequel R₁ est éventuellement protégé par un groupe protecteur, avec une amine de formule (III) : 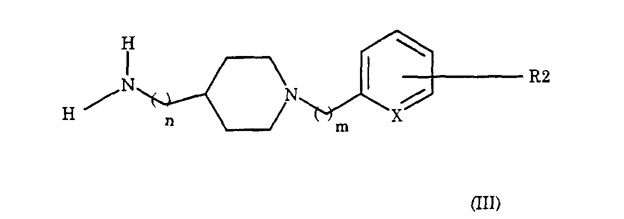 où m, n, R₂ et X sont tels que indiqués ci-dessus, en éliminant le groupe protecteur éventuellement présent et en transformant le produit de formule (I) ainsi obtenu en un de ses sels ou solvates.

Alternativement, lorsque A représente un groupe (b) et R₁ est un groupe NH₂, les composés de formule (I) sont préparés de préférence par condensation d'une amine de formule (III) avec un produit de formule (II) où A' est le groupe (b) et R₁ est un groupe de formule : 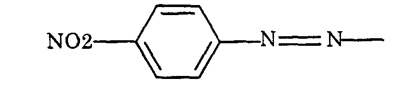 et en soumettant le produit de formule (IV) ainsi obtenu : 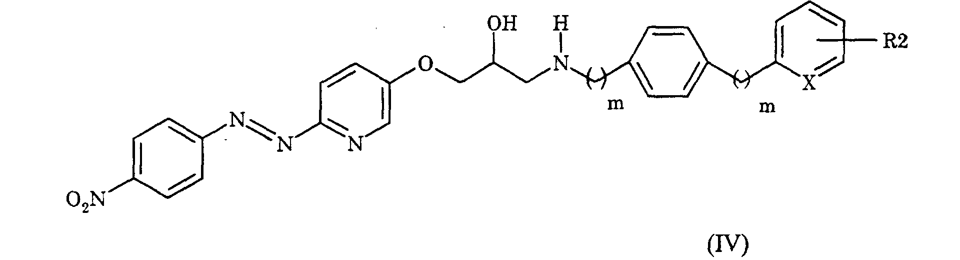 à une réaction d'hydrogenation pour transformer le groupe 4-nitrophényldiazenyle en groupe amino, et éventuellement on transforme le produit de formule (I) ainsi obtenu en un de ses sels ou solvates.

Lorsque A est un groupe de formule (a) et Y₁ et Y₂ représentent un atome d'azote il est également possible de préparer les composés de formule (I) en traitant un composé de formule (V) : 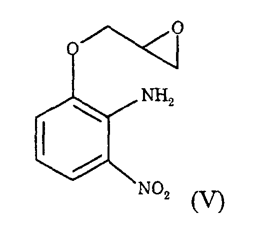 avec une amine de formule (III), en réduisant le groupe nitro du produit de formule (VI) ainsi obtenu: 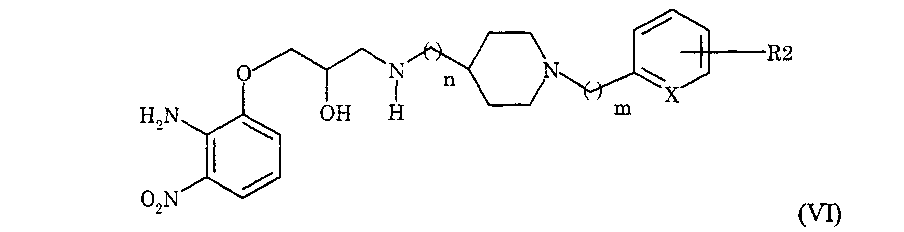 et en traitant le produit de formule (VII) ainsi obtenu : 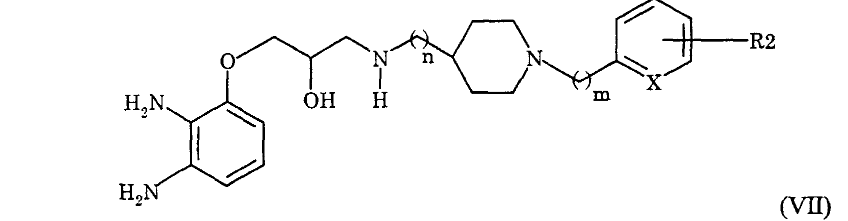 avec un agent de carbonylation, à savoir un agent capable d'insérer dans la molécule un groupe carbonyle, tel que par exemple le carbonyldiimidazole ou le phosgène, pour obtenir le produit final, qui peut éventuellement être transformé en un de ses sels ou solvates.

La réaction de réduction du groupe nitro en amino peut être conduite par exemple par hydrogénation catalytique, comme solvant de réaction on peut utiliser par exemple un solvant polaire protique comme l'eau, l'acide acétique, un alcool tel que l'éthanol, le méthanol, l'isopropanol, un ester tel que l'acétate d'éthyle, un éther linéaire ou cyclique tel que le tétrahydrofuranne ou le dioxane, ou encore un solvant aromatique tel que le benzène ou le toluène.

La réaction de cyclisation est de préférence conduite à l'aide du carbonyldiimidazole, dans un solvant inerte tel que le tétrahydrofuranne ou un éther linéaire, à une température comprise entre la température ambiante et la température de reflux du solvant choisi.

La réaction entre les époxydes et l'amine (III) est réalisée dans un solvant organique, tel qu'un alcool inférieur comme le méthanol, l'éthanol ou l'isopropanol; le diméthylsulfoxyde; un éther linéaire ou cyclique; un amide comme le diméthylformamide ou le diméthylacétamide, et en utilisant des quantités au moins équimoléculaires des réactifs, éventuellement un faible excès d'amine.

La température de la réaction est comprise entre la température ambiante et la température de reflux du solvant choisi.

Les composés de formule (II) dans lesquels A' ; est un groupe (a) peuvent être préparés selon le procédé général décrit dans le schéma III de WO97/10825 ou selon le brevet DE 2700193.

Les composés de formule (II) où A' est un groupe (b) peuvent être préparés selon le procédé général décrit dans EP 0 611 003.

Les amines de formule (III) peuvent être préparées par réaction des synthons convenables de formule (VIII) : 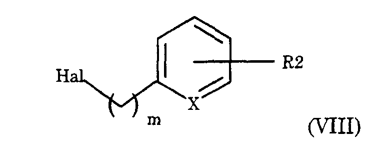 où Hal représente un halogène et R₂, m et X sont tels que définis ci-dessus, avec une pipéridine de formule (IX) : 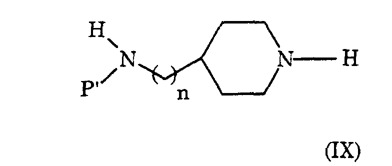 où n est tel que défini ci-dessus et P' représente un groupe protecteur, dans un solvant organique, en présence d'une base, suivie par l'élimination du groupe P' des composés de formule (X) ainsi obtenus :

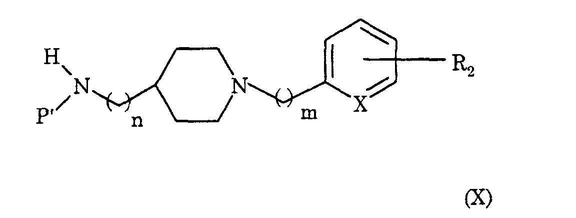

Comme solvant de réaction, on peut utiliser par exemple le diméthylformamide, la pyridine, le diméthylsulfoxyde, un éther linéaire ou cyclique ou un solvant chloré tel que le dichlorométhane.

Comme base on peut utiliser par exemple un hydroxyde alcalin, un carbonate alcalin tel que le carbonate de potassium ou une amine tertiaire telle que la triéthylamine.

La réaction de condensation ci-dessus est complète en quelques heures, normalement en 2 à 12 heures.

La température de réaction est comprise entre la température ambiante et la température de reflux du solvant choisi.

Comme groupes protecteurs P', on peut utiliser les groupes acyles, tel que formyle, acétyle, propionyle, phénylacétyle phénoxyacétyle et similaires ; un groupe alcoxycarbonyle tel que tert-butoxycarbonyle et similaires ; un groupe alcoxycarbonyle tel que méthoxypropionyle et similaires ; un groupe alcoxycarbonyle substitué tel que monochlorométhylcarbonyle, dichlorométhylcarbonyle, trichlorométhylcarbonyle, trichloroéthylcarbonyle, trichloropropylcarbonyle, trifluorométhylcarbonyle et similaires ; un groupe arylalkoxycarbonyle substitué tel que 4-nitrobenzyloxycarbonyle et similaires ; un groupe benzyle ; un groupe benzyle substitué ; un groupe diphénylméthyle éventuellement substitué ; un groupe trityle éventuellement substitué, tel que 4-méthoxyphényldiphénylméthyle ou di-(4-méthoxyphényl)phénylméthyle ; un groupe silylant tel que triméthylsilyle ou éthyldiméthylsilyle ou tert-butyldiméthylsilyle et similaires.

Lesdits groupes protecteurs peuvent être éliminés selon les méthodes conventionnelles par exemple par réduction ou hydrolyse. Une description plus détaillée de ces groupes amino-protecteurs ainsi que les méthodes pour leur préparation et leur élimination sont données par exemple par T.W. Greene, "Protective Groups in Organic Synthesis", John Wiley & Sons, 1981 et par J.F.W. McOmie, "Protective Groups in Organic Chemistry", Plenum Press, 1973.

L'elimination de ces groupes protecteurs est effectuée selon les méthodes habituelles décrites pour le groupe protecteur choisi; dans le cas du *tert*-butoxycarbonyle par l'élimination, le clivage est normalement effectuée par hydrolyse acide.

Les composés de formule (I) ont montré une activité puissante vis-à-vis des récepteurs β₃-adrenergiques. En outre, ces composés sont très peu toxiques ; notamment leur toxicité aigue est compatible avec leur utilisation comme médicaments pour le traitement de maladies dans lesquelles les composés ayant une affinité pour le récepteur β₃ trouvent leur application.

L'activité des composés de la présente invention vis-à-vis de l'activité β₃ a été mise en évidence à l'aide d'essais in vitro sur le colon humain selon la méthode décrite dans EP-B-436435 et dans T. Croci et al, Br. J. Pharmacol., 1997, 122: 139P.

Plus particulièrement, on a constaté que les composés de formule (I) sont beaucoup plus actifs sur le colon isolé que sur l'oreillette et sur la trachée.

Ces propriétés surprenantes des composés de formule (I) permettent d'envisager leur utilisation comme médicaments à action β₃.

Les composés de formule (I), ainsi que leurs sels pharmaceutiquement acceptables, peuvent donc être indiqués par exemple dans le traitement des maladies gastro-intestinales telles que le syndrôme du colon irritable, comme modulateurs de la motricité intestinale, comme lipolytiques, agents anti-obésité, anti-diabétiques, psychotropes, anti-glaucomateux, cicatrisants, anti-dépressants, comme tocolytiques pour prévenir ou retarder les accouchement précoces, pour le traitement et/ou la prophylaxie de la dysménorrhée.

L'utilisation des composés de formule (I) ci dessus, ainsi que celle de leurs sels et solvates pharmaceutiquement acceptables pour la préparation de médicaments ci-dessus, constitue un aspect ultérieur de la présente invention.

Pour une telle utilisation, on administre aux mammifères qui nécessitent un tel traitement une quantité efficace d'un composé de formule (I) ou d'un de ses sels et solvates pharmaceutiquement acceptables.

Les composés de formule (I) ci-dessus et leurs sels et solvates pharmaceutiquement acceptables peuvent être utilisés à des doses journalières de 0,01 à 20 mg par kilo de poids corporel du mammifère à traiter, de préférence à des doses journalières de 0,1 à 10 mg/kg. Chez l'être humain, la dose peut varier de préférence de 0,5 mg à 1500 mg par jour, notamment de 2,5 à 500 mg selon l'âge du sujet à traiter, le type de traitement, prophylactique ou curatif, et la gravité de l'affection. Les composés de formule (I) sont généralement administrés en unité de dosage de 0,1 à 500 mg, de préférence de 0,5 à 100 mg de principe actif, une à cinq fois par jour.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I) ci-dessus ou un de ses sels et solvates pharmaceutiquement acceptables.

Dans les compositions pharmaceutiques de la présente invention pour l'administration orale, sublinguale, sous-cutanée, intramusculaire, intraveineuse, topique, transdermique ou rectale, les principes actifs de formule CI) ci-dessus, leurs sels et solvates pharmaceutiquement acceptables peuvent être administrés sous formes unitaires d'administration, en mélange avec des supports pharmaceutiques classiques, aux animaux et aux êtres humains, pour le traitement des affections susdites. Les formes unitaires d'administration appropriées comprennent les formes par voie orale telles que les comprimés, les gélules, les poudres, les granulés et les solutions ou suspensions orales, les formes d'administration sublinguale et buccale, les formes d'administration sous-cutanée, intramusculaire ou intraveineuse, les formes d'administration locale et les formes d'administration rectale.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Les poudres ou les granulés dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration locale, on mélange le principe actif dans un excipient pour la préparation de crèmes ou onguents ou on le dissout dans un véhicule pour l'administration intraoculaire, par exemple sous forme de collyre.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Selon un autre de ses aspects, la présente invention concerne une méthode de traitement des pathologies qui sont améliorées par une action β₃-agoniste, qui consiste à administrer un composé de formule (I) ou l'un de ses sels ou solvates pharmaceutiquement acceptables.

Les composés de formule (I), notamment les composés (I) marqués par un isotope, peuvent aussi être utilisés comme outils de laboratoire dans des essais biochimiques.

Les composés de formule (I) se lient au récepteur β₃-adrénergique. On peut donc utiliser ces composés dans un essai ordinaire de liaison ("binding"), dans lequel on emploie un tissu organique où ce récepteur est particulièrement abondant, et on mesure la quantité de composé (I) déplacé par un composé test, pour évaluer l'affinité dudit composé vis-à-vis des sites de liaison de ce récepteur particulier.

Un autre objet spécifique de la présente invention est donc un réactif utilisable dans les essais biochimiques, qui comprend au moins un composé de formule (I) convenablement marqué.

Les exemples qui suivent illustrent l'invention.

### EXEMPLE 1

### 3-[1-(5-éthoxycarbonylpyrid-2-yl)-4-pipéridinylamino]-1-11,2-dibydro-2-oxo-benzimidazol-4-yloxy]-2-propanol.

### a) 4-tert-butoxycarbonylamino-pipéridine.

On mélange à la température ambiante pendant 2 heures 25 g (0,13 mole) de 4-amino-1-benzylpipéridine, 36,2 ml (0,26 mole) de triéthylamine et 31,2 g (0,143 mole) de di*-tert*-butyl-dicarbonate dans 200 ml de diméthylformamide. On verse le mélange dans de l'eau, on extrait à l'acétate d'éthyle, on lave à l'eau et on cristallise le produit ainsi obtenu dans 200 ml d'éther isopropylique. On obtient 33 g de 1-benzyl-4-*tert*-butoxycarbonylamino-pipéridine qu'on hydrogène dans un mélange de 200 ml d'éthanol et 100 ml de tétrahydrofuranne en présence de 3 g de Pd/C à 10%. Après filtration du catalyseur, on isole le composé du titre. P.f. :157-160°C.

### b) 4-tert-butoxycarbonylamino-1-(5-éthoxycarbonylpyrid-2-yl)-pipéridine.

On chauffe à 80°C pendant 18 heures un mélange du produit préparé ci-dessus, de la triéthylamine et de l'ester éthylique de l'acide 6-chloronicotinique. Après refroidissement, on ajoute de l'eau, on extrait l'acétate d'éthyle, on sèche la phase organique sur du sulfate de sodium et on évapore le solvant sous pression réduite. On obtient le composé du titre. P.f. 140-142°C.

### c) 4-amino-1-(5-éthoxycarbonylpyrid-2-yl)-pipéridine (dichlorhydrate hydraté).

On dissout le produit de l'étape b) dans de l'acétate d'éthyle et on ajoute une solution 3N d'acide chlorhydrique dans de l'acétate d'éthyle et on laisse agiter à la température ambiante pendant 10 heures. On filtre et on lave à l'acétone. On obtient le produit du titre. P.f. :148-150°C.

### d) 2 amino-3-nitro-1-(2,3-époxypropoxy)-benzène.

On mélange 21,7g (0,095 mole) de glycidyl-tosylate, 10 g (0,0475 mole) de 2-amino-3-nitrophénol, 6,5 g de K₂CO₃ broyé dans l'acétone et on chauffe au reflux pendant 18 heures. On filtre et on évapore le solvant sous pression réduite. On purifie le brut de réaction par flash chromatographie en éluant par un mélange hexane/acétate d'éthyle =9/1. On obtient le composé du titre. P.f. :76°-78°C.

### e) 3-[1-(5-éthoxycarbonylpyrid-2-yl)-4-pipéridinylamino]-1-(2-amino-3-nitrophenoxy)-2-propanol.

On mélange 1 g (0,00475 mole) du composé obtenu dans l'étape précédente avec 1,53 g (0,00475 mole) de 4-amino-1-(5-éthoxycarbonylpyrid-2-yl)-pipéridine (étape c, base) dans 50 ml d'éthanol. On chauffe à reflux pendant une nuit et on évapore sous pression réduite. Le brut de réaction est purifié par flash chromatographie en éluant par un mélange acétate d'éthyle/éthanol=9/1. On obtient le composé du titre.
P.f. : 140-142°C.

### f) 3-[1-(5-éthoxycarbonylpyrid-2-yl)-4-pipéridinylamino]-1-(2,3 diaminophenoxy)-2-propanol

1,71 g (0,0037 mole) du composé de l'étape précédente sont hydrogénés à température ambiante dans 120 ml d'éthanol en présence de 0,8 g de Pd/C à 5%. Après avoir filtré et évaporé le solvant on purifie le brut de réaction par flash chromatographie en éluant par un mélange acétate d'éthyle/éthanol =7/3. On obtient le composé du titre.

### g) 3-[1-(5-éthoxycarbonylpyrid-2-yl)-4-pipéridinylamino]-1-[1,2-dihydro-2-oxo-benzimidazol-4-yloxyl-2-propanol.

Le produit obtenu dans l'étape précédente est placé sous agitation à température ambiante pendant une nuit avec 0,44 g de N,N-carbonyldiimidazole (0,027 mole) dans 50 ml de THF. On évapore le solvant sous pression réduite, on ajoute de l'acétate d'éthyle et on lave à l'eau. Après déshydratation et évaporation du solvant, on purifie par chromatographie une première fois en éluant par un mélange chlorure de méthylene/méthanol = 8/2 et une deuxième fois en éluant par un mélange méthanol/acetate d'éthyle = 8/2. On obtient le produit du titre P.f. :191°-193°C.

### EXEMPLE 2

### 3-[1-(5-éthoxycarbonylpyrid-2-yl)-4-pipéridinylamino]-1-[2-amino-pyrid-5-yloxy]-2-propanol.

### a) 2-[2-(4-nitrophényl)-diazényl]-5-(2,3-époxypropoxy)-pyridine

A une solution contenant 1,82 g de 5-hydroxy-2-(2-(4-nitrophenyl)diazényl)pyridine (0,01043 mole), preparée selon le procédé décrit en J. Am. Chem. Soc., 1959, 81, 6049, 0,692 ml de 2,3-époxypropanol (0,01043 mole) et 2,74 g de Ph₃P (0,01043 mole) dans 18 ml de DMF on ajoute à 0°C, sous atmosphère d'azote, 1,64 ml de diéthylazodicarboxylate (0,01043 mole). On laisse réagir pendant une heure à 0°C et puis pour 40 heures à la température ambiante sous agitation. On ajoute de l'eau, on extrait à l'acétate d'éthyle, on lave et on évapore le solvant. Le brut de réaction est purifié par chromatographie en éluant par un mélange CH₂Cl₂/CH₃OH =100/2. On obtient le produit du titre. P.f:150-152°C (déc).

### b) 3-[1-(5-éthoxycarbonylpyrid-2-yl)-4-pipéridinylamino]-1-[2-(2-(4-nitrophényl)-diazényl)-pyrid-5-yloxy]-2-propanol

On chauffe au reflux pendant 7 heures, une solution de 1,15 g (0,00383 mole) du produit obtenu dans l'étape a) et 1,05 g (0,00421 mole) de 1-(5-éthoxycarbonylpyrid-2-yl)-4-pipéridylamine dans 20 ml d'éthanol. On filtre, on sèche et on cristallise dans une solution d'éthanol et CH₂Cl₂. On obtient le produit du titre. P.f : 172°C.

### c) 3-[1-(5-éthoxycarbonylpyrid-2-yl)-4-pipéridinylamino]-1-[2-amino-pyrid-5-yloxy]-2-propanol

On dissout 1,37 g (0,002509 mole) du produit de l'étape c) avec 0,16 g de Pd/C dans 30 ml d'éthanol et 2 ml de CH₃COOH (d= 1.049, 0,0347 mole). On hydrogène pendant 9 heures sous agitation à une température comprise entre 15 et 20 °C. Le brut de réaction est filtré sur célite et lavé à l'éthanol. On évapore le solvant et on ajoute 30 ml d'une solution saturée en NaHCO₃, 5 ml de NaOH 1N et on extrait à l'acétate d'éthyle. On évapore le solvant et on purifie le brut de réaction par chromatographie en éluant par un mélange CH₂Cₗ₂/CH₃OH/NH₄OH = 95/5/0,5 et ensuite 90/10/1.

On obtient le produit du titre. P.f : 120-122°C.

### EXEMPLE 3

### 3-[1-(5-éthoxycarbonylpyrid-2-yl)-4-pipéridinylamino]-1-(1,2-dihydro-2-oxo-benzimidazol-4-yloxy)-(2S)-2-propanol.

### a) 2-amino-3-nitro-1-((2S)-2,3-époxypropoxy)-benzène.

On mélange 5 g (0,032 mole) de 2-amino-3-nitrophénol, 8 g (0,032 mole) de (S)(+)glycidyl-3-nitrobenzènesulfonate, 8,9 g de K₂CO₃ dans 80 ml d'acétone et on chuffe au reflux pendant 18 heures. On filtre, on évapore le solvant sous pression réduite, on purifie le brut par flash chromatographie en éluant avec un mélange cyclohexane/acétate d'éthyle = 7/3. Le produit solide obtenu est trituré dans l'éther éthylique et on obtient 5,67 g du produit du titre. P.f. :107-109°. αD=+28,1(c=0,5%, MeOH).

### b) 3-[1-(5-éthoxycarbonylpyrid-2-yl)-4-pipéridinidylamino]-1-(2-amino-3-nitrophénoxy)-(2S)-2-propanol.

En suivant la méthode de l'exemple 1 e), mais en partant du composé préparé ci-dessus, on obtient le produit du titre. αD=+18,3 (c=1%, MeOH).

### c) 3-[1-(5-éthoxycarbonylpyrid-2-yl)-4-pipéridinidylamino]-1-(2,3-diamino-phénoxy)-(2S)-2-propanol.

En suivant la méthode de l'exemple 1 f), mais en partant du composé préparé ci-dessus, on obtient le produit du titre.

### d) 3-[1-(5-éthoxycarbonylpyrid-2-yl)-4-pipéridinylamino]-1-(1,2-dihydro-2-oxo-benzimidazol-4-yloxy)-(2S)-2-propanol.

On met 0,3 g (0,061 mole) du produit obtenu dans l'étape c) sous agitation à température ambiante pendant 4 heures dans 4 ml de toluène et 4 ml d'eau avec 0,059 (0,488 mole) chloroformiate de trichlorométhyle. On filtre, on dissout le produit dans l'éthanol et l'ammoniaque, on évapore le solvant, on purifie par flash chromatographie en éluant par un mélange chlorure de méthylène/méthanol/ammoniaque=90/10/1 et on obtient le produit du titre. Solide amorphe.

### Spectre RMN à 200 MHz (¹H) et 50 MHz (¹³C):

¹H NMR(aromatiques) - ν_{TMS}(CDCL₃, ppm): 6.3-6.6 (3H, m), 6.6-6.9 (1H, m); 7,91 (1H, dd, J₁ 9 Hz, J₂ 2 Hz), 8.72 (1H, d, J 2 Hz).
¹³C NMR - ν_{TMS}(CDCL₃, ppm): 14.3, 31.6, 31.7, 43.5, 48.6, 55.1, 60.3, 69.0, 71.3, 103.2, 105.0, 114.3, 118.9, 121.7, 129.9, 138.3, 143.2, 150.9, 156.7, 160.2, 165.9.

### EXEMPLE 4

### 3-[1-(4-éthoxycarbonylphényl)-4-pipéridinylamino]-1-(1,2-dihydro-2-oxobenzimidazol-4-yloxy)-(2S)-2-propanol.

### a) 4-tert-butoxycarbonylamino-pipéridine.

On mélange à la température ambiante pendant 2 heures 25 g (0,13 mole) de 4-amino-1-benzylpipéridine, 36,2 ml (0,26 mole) de triéthylamine et 31,2 g (0,143 mole) de dicarbonate de di-*tert*-butyle dans 200 ml de diméthylformamide. On verse le mélange dans de l'eau, on extrait à l'acétate d'éthyle, on lave à l'eau et on cristallise le produit ainsi obtenu dans 200 ml d'éther isopropylique. On obtient 33 g de 1-benzyl-4-*tert*-butoxycarbonylamino-pipéridine qu'on hydrogène dans un mélange de 200 ml d'éthanol et 100 ml de tétrahydrofurane en présence de 3 g de Pd/C à 10%. Après filtration du catalyseur, on isole le composé du titre.
P.f. :157-160°C.

### b) 4-tert-butoxycarbonylamino-1-(4-éthoxycarbonylphenyl)-pipéridine

On chauffe à 80°C pendant 55 heures 21,6 g (0,10 mole) du produit obtenu ci-dessus avec 9,06 g (0,01 mole) de (4-éthoxycarbonyl-1-fluoro)benzène et 14,9 g de K₂CO₃ dans 200 ml de diméthylformamide. On filtre le K₂CO₃, on verse la solution dans l'eau, , on extrait à l'acétate d'éthyle et on évapore le solvant. Le brut de réaction est purifié par flash chromatographie en éluant par un mélange cyclohexane/acétate d'éthyle = 8:2. On obtient le produit du titre qui est cristallisé dans l'acétate d'éthyle. P.f.=138°-140°

### c) 4-amino-1-(4-éthoxycarbonylphényl)-pipéridine (chlorhydrate)

On dissout 9,74 g (0,023 mole) du produit obtenu dans c) dans 60 ml d'acétate d'éthyle et on ajoute 80 ml d'une solution de HCl 3N dans l'acétate d'éthyle. On chauffe à reflux pendant 5 heures, on évapore le solvant, on ajoute de l'acétone et on filtre. On obtient le produit du titre qui cristallise dans l'éthanol. P.f. :240-242°.

### d) 3-[1-(4-éthoxycarbonylphényl)-4-pipéridinylamino]-1-(2-amino-3-nitrophenoxy)-(2S)-propanol.

En suivant la méthode de l'exemple 1 e), mais en utilisant 1 g (0,0040 mole) du composé obtenu précédemment (base) et 0,85 g (0,0040 mole) de l'époxyde de l'exemple 3 a), on obtient, après purification par flash chromatographie avec un mélange chlorure de méthylène/méthanol=95/5, 1,24 g du produit du titre.
P.f. :112-114°.

### e) 3-[1-(4-éthoxycarbonylphényl)4-pipéridinylamino]-1-(2,3-diamino-phenoxy)-(2S)-propanol.

En suivant la méthode de l'exemple 1 f), mais en partant de 1,2 g (0,0026 mole) du composé obtenu précédemment, on obtient 1,2 g du produit du titre.

### f) 3-[1-(4-éthoxycarbonylphényl)-4-pipéridinylamino]-1-(1,2-dihydro-2-oxobenzimidazol-4-oxy)-(2S)-propanol

Le produit obtenu dans l'étape précédente est placé sous agitation à température ambiante pendant 4 heures avec 0,57 g (0,0029 mole) de chloroformiate de trichlorométhyle dans 20 ml de toluène et 20 ml de tétrahydrofurane (THF). On évapore le solvant sous pression réduite, on ajoute de l'acétate d'éthyle, de l'ammoniaque et on lave à l'eau. On sèche la phase organique sur sulfate de sodium, on évapore le solvant et on purifie par flash chromatographie en éluant par un mélange de méthanot/acétate d'éthyle=8/2. On obtient 0,72 g du produit du titre.
P.f. :188-190°. α365=+41,5 (c=1%, MeOH).

### EXEMPLE 5

### 3-[1-(4-nbutylaminocarbonylphényl)-4-pipéridinylamino]-1-(1,2-dihydro-2-oxobenzimidazol-4-oxy)-(2S)-propanol.

### a) 4-tert-butoxycarbonylamino-1-(4-hydroxycarbonylphenyl)-pipéridine

On dissout 2,19 g (0,0063 mole) du produit de l'exemple 4 b) dans 30 ml de THF et 20 ml d'eau, et on ajoute 12,6 ml (0,0126 mole) de NaOH 1N. Après 24 heures à température ambiante,on ajoute de l'acide acétique jusqu'à pH 7, on évapore le solvant, on triture le solide ainsi obtenu dans l'eau, on filtre et on cristallise dans l'éthanol. On obtient 1,32 g du composé du titre. P.f. >300°.

### b) 4-tert-butoxycarbonylamino-1-(4-nbutylaminocarbonylphenyl)-pipéridine

On mélange 2,5 g (0,0078 mole) du produit de l'étape précédente, 3,45 g (0,0078 mole) de (benzotriazol-1-yloxy)tris(diméthylamino)phosphonium hexafluorophosphate, 0,57 g (0,0078 mole) de *n*-butylamine et 1,7 ml (0,012 mole) de triéthylamine dans 80 ml de chlorure de méthylène et on chauffe à 40° pendant 8 heures. On évapore le solvant et on ajoute de l'acétate d'éthyle et une solution saturée de NaHCO₃. On obtient un solide en suspension qu'on filtre et lave à l'eau puis à l'acétate d'éthyle. On cristallise de 50 ml d'isopropanol et on obtient 2,14 g du produit du titre. P.f. :208-210°.

### c) 4-amino-1-(4-nbutylaminocarbonylphenyl)-pipéridine (chlorhydrate dihydraté)

En suivant la méthode de l'exemple 4 c), mais en utilisant le composéde l'étape précédente comme produit de départ, on obtient, après cristallisation de l'éthanol, 1,66 g du produit du titre. P.f. :231-235°.

### d) 3-[1-(4-nbutylaminocarbonylphényl)-4-pipéridinylamino]-1-(2-amino-3-nitrophenoxy)-(2S)-propanol.

En utilisant 0,49 g (0,0018 mole) du produit de l'exemple précédent (base),0,4 g (0,0019 mole) de l'époxyde de l'exemple 3 a) dans 20 ml d'éthanol et en suivant la méthode de l'exemple 1e), on obtient, après purification par flash chromatographie avec un mélange méthanol/ammoniaque= 100/1, 0,57g du composé du titre. P.f.=68-70°.

### e) 3-[1-(4-nbutylaminocarbonylphényl)4-pipéridinylamino]-1-(2,3-diamino-phenoxy)-(2S)-propanol.

En opérant comme dans l'exemple 1 f), mais en utilisant le composé obtenu précédemment comme produit de départ, on obtient 0,52 g du produit du titre.

### f) 3-[1-(4-nbutylaminocarbonylphényl)-4-pipéridinylamino]-1-{1,2-dihydro-2-oxobenzimidazol-4-oxy)-(2S)-propanol.

Le produit obtenu dans l'étape précédente est placé sous agitation à température ambiante pendant 4 heures avec 0,23 g(0,0012 mole) de chloroformiate de trichlorométhyle dans 20 ml de THF et 4 ml de chlorure de méthylène. On évapore le solvant sous pression réduite, on ajoute de l'acétate d'éthyle, de l'ammoniaque , et on lave à l'eau. On sèche la phase organique sur sulfate de sodium, on évapore le solvant et on purifie par flash chromatographir en éluant par un mélange méthanol/acétate d'éthyle=3/7. On obtient 0,052g du composé du titre. P.f. :82-84°.

## Revendications

1. Composé de formule (I) : 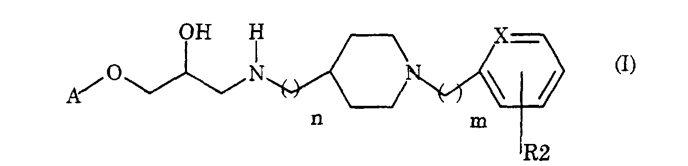 où
X est N ou CH ;
A représente un groupe de formule (a) ou (b) 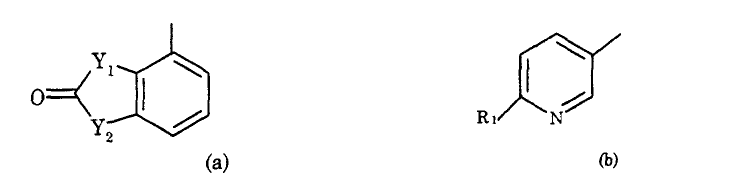
R₁ représente un atome d'hydrogène, ou un groupe -NH₂, -NR₃R₄, -NR₃CO(C₁-C₄)Alk ou -NR₃SO₂(C₁-C₄)Alk;
R₂ représente un atome d'hydrogène ou d'halogène, un groupe (C₁-C₄)Alk, (C₁-C₄)alcoxy, -COOH, -COO(C₁-C₄)Alk, -CN, -CONR₃R₄, -NO₂, -SO₂NR₃R₄, -NHSO₂(C₁-C₄)Alk;
m et n représentent chacun 0, 1 ou 2 ;
R₃ et R₄ représentent chacun un atome d'hydrogène ou un groupe (C₁-C₄)Alk;
Y₁ et Y₂ représentent chacun NH ou O; et leurs sels ou solvates.

2. Composés selon la revendication 1, où X représente CH.

3. Composés selon la revendication 1, où X représente un atome d'azote et le groupe R₂ est dans la position 5.

4. Composés selon la revendication 1 où le groupe (C₁-C₄)Alk est un groupe méthyle ou éthyle.

5. Composés selon la revendication 1 où R₂ est choisi parmi -COOH, -COO(C₁-C₄)Alk, -CN, -NO₂,-CONR₃R₄ et -NHSO₂-(C1-C₄)Alk.

6. Le 3-[1-(5-éthoxycarbonylpyrid-2-yl)-4-pipéridinylamino]-1-(1,2-dihydro-2-oxo-benzimidazol-4-yloxy)-2-propanol et ses sels ou solvates selon la revendication 1.

7. Le 3-[1-(5-éthoxycarbonylpyrid-2-yl)-4-pipéridinylaininoj-l-[2-aminopyrid-5-yloxy]-2-propanol et ses sels ou solvates selon la revendication 1.

8. Procédé pour la préparation des composés de la revendication 1 **caractérisé en ce qu'**on fait réagir un époxyde de formule (II) : 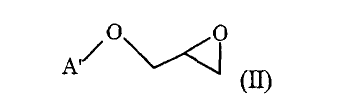 dans laquelle A' représente le groupe (a) ou le groupe (b) dans lequel R₁ est éventuellement protégé, où (a), (b) et R₁ sont tels que définis dans la revendication 1, avec une amine de formule (III) 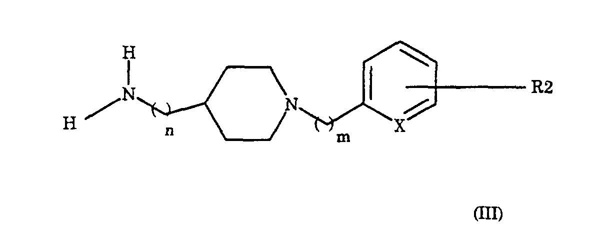 où m, n, R₂ et X sont tels que indiqués ci-dessus, on elimine le groupe protecteur éventuellement présent et éventuellement on transforme le produit de formule (I) ainsi obtenu en un de ses sels ou solvates.

9. Procédé pour la préparation d'un composé de la revendication 1 où A représente un groupe (b) et R₁ est un groupe NH₂ **caractérisé en ce qu'**on fait réagir un produit de formule (II) tel que défini dans la revendication 8 où A' est le groupe (b) et R₁ est un groupe de formule : 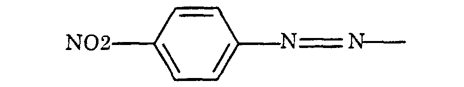 avec une amine de formule III et on soumet le produit de formule IV ainsi obtenu : 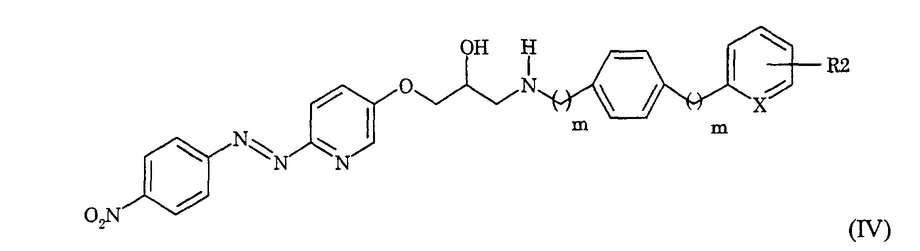 à une réaction d'hydrogénation pour transformer le groupe 4-nitrophényldiazenyle en groupe amino et éventuellement on transforme le produit de formule (I) ainsi obtenu en un de ses sels ou solvates.

10. Procédé pour la préparation des composés de la revendication 1 où A représente le groupe (a) et Y₁ et Y₂ représentent un atome d'azote **caractérisé en ce qu'**on fait réagir un composé de formule (V) : 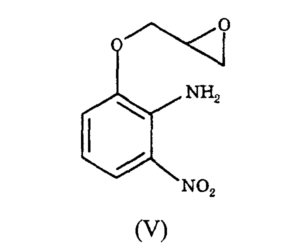 avec un composé de formule (III) tels que défini dans la revendication 8, on réduit le groupe nitro du produit de formule (VI) ainsi obtenu : 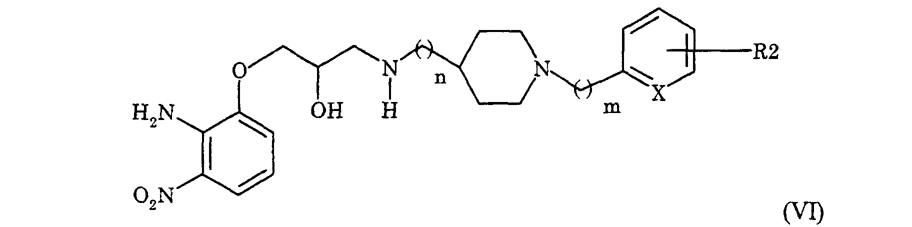 on traite le produit de formule (VII) ainsi obtenu : 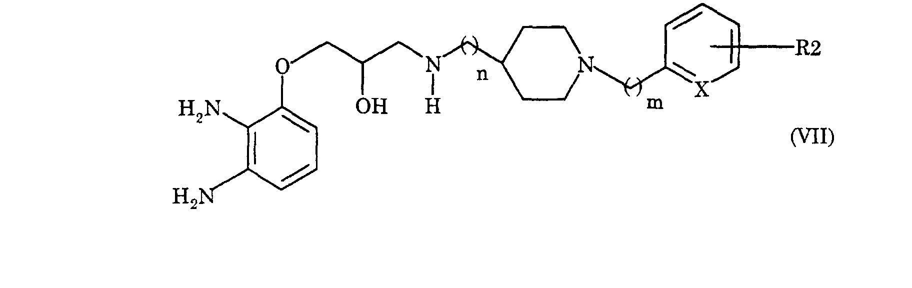 avec un agent de carbonylation, on isole le produit de formule (I) ansi obtenu et éventuellement on le transforme en un de ses sels ou solvates.

11. Procédé selon la revendication 10 **caractérisé en ce que** l'agent de carbonylation est choisi parmi le carbonyldiimidazole et le phosgène.

12. Composition pharmaceutique comprenant au moins un composé de la revendication 1 en tant que principe actif.

13. Utilisation d'un composé selon la revendication 1 pour la préparation de médicaments indiqués dans la syndrome du colon irritable, ou à action modulatrice de la motilité intestinale, lipolytique, anti-obésité, anti-diabétique, psychotrope, anti-glaucomateuse, cicatrisante, anti-dépressive, comme tocolytiques pour prévenir ou retarder les accouchement précoces, pour le traitement et/ou la prophylaxie de la dysménorrhée.

## Patentansprüche

1. Verbindung der Formel (I): 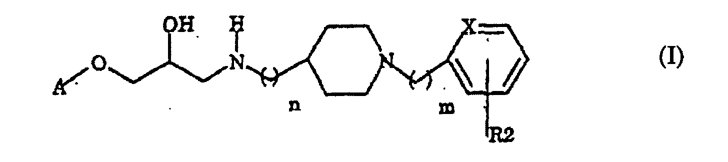 in der
X N oder CH bedeutet;
A eine Gruppe der Formel (a) oder (b) bedeutet: 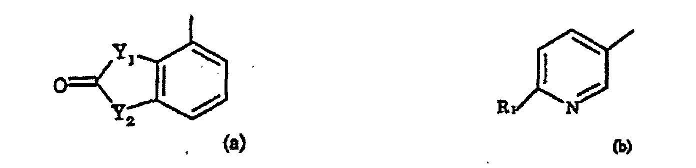
R₁ ein Wasserstoffatom oder eine Grupe -NH₂, -NR₃R₄, -NR₃CO(C₁-C₄)Alk oder -NR₃SO₂(C₁-C₄)Alk bedeutet;
R₂ ein Wasserstoffatom, ein Halogenatom oder eine Gruppe (C₁-C₄)Alk, (C₁-C₄)Alkoxy, -COOH, -COO(C₁-C₄)Alk, -CN, -CONR₃R₄, -NO₂, -SO₂NR₃R₄ oder -NHSO₂(C₁-C₄)Alk darstellt;
m und n jeweils 0, 1 oder 2 bedeuten;
R₃ und R₄ jeweils ein Wasserstoffatom oder eine Gruppe (C₁-C₄)Alk darstellen;
Y₁ und Y₂ jeweils NH oder O bedeuten;
und deren Salze oder Solvate.

2. Verbindungen nach Anspruch 1, worin X CH bedeutet.

3. Verbindungen nach Anspruch 1, worin X ein Stickstoffatom bedeutet und die Gruppe R₂ in der 5-Stellung steht.

4. Verbindungen nach Anspruch 1, worin die Gruppe (C₁-C₄)Alk eine Methyl-oder eine Ethylgruppe darstellt.

5. Verbindungen nach Anspruch 1, worin R₂ ausgewählt ist aus -COOH, -COO((C₁-C₄)Alk, -CN, -NO₂, -CONR₃R₄ und -NHSO₂-(C₁-C₄)Alk.

6. 3-[1-(5-Ethoxycarbonylpyrid-2-yl)-4-piperidinylamino]-1-(1,2-dihydro-2-oxo-benzimidazol-4-yloxy)-2-propanol und dessen Salze oder Solvate nach Anspruch 1.

7. 3-[1-(5-Ethoxycarbonylpyrid-2-yl)-4-piperidinylamino)-1-[2-aminopyrid-5-yloxy]-2-propanol und dessen Salze oder Solvate nach Anspruch 1.

8. Verfahren zur Herstellung der Verbindungen nach Anspruch 1, **dadurch gekennzeichnet, daß** man ein Epoxid der Formel (II): 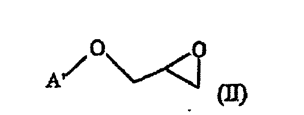 in der A' die Gruppe (a) oder die Gruppe (b) bedeutet, in der R₁ gegebenenfalls geschützt ist, worin (a), (b) und R₁ die in Anspruch 1 angegebenen Bedeutungen besitzen,
mit einem Amin der Formel (III) 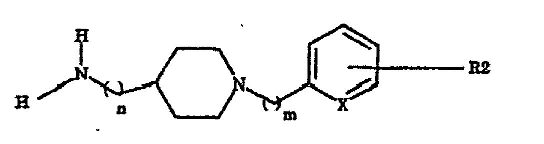 in der m, n, R₂ und X die oben angegebenen Bedeutungen besitzen, umsetzt und die gegebenenfalls vorhandene Schutzgruppe entfernt und das in dieser Weise erhaltene Produkt der Formel (I) in eines seiner Salze oder Solvate umwandelt.

9. Verfahren zur Herstellung einer Verbindung nach Anspruch 1, worin A eine Gruppe (b) bedeutet und R₁ eine Gruppe NH₂ darstellt, **dadurch gekennzeichnet, daß** man ein Produkt der Formel (II), wie es in Anspruch 8 definiert ist, worin a' die Gruppe (b) bedeutet und R₁ eine Gruppe der Formel: 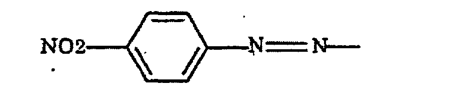 darstellt,
mit einem Amin der Formel III umsetzt und das in dieser Weise erhaltene Produkt der Formel IV: 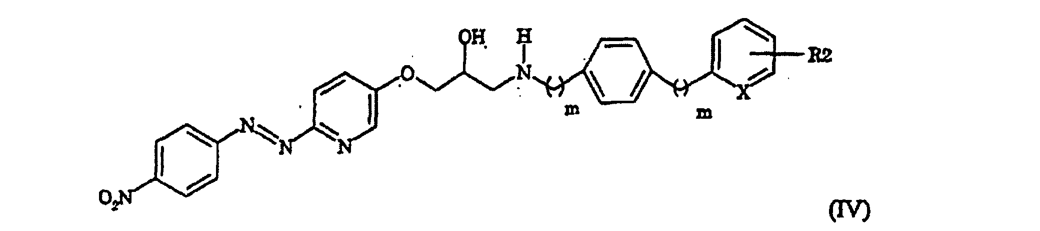 einer Hydrierungsreaktion unterwirft zur Umwandlung der 4-Nitrophenyldiazenylgruppe in die Aminogruppe und man gegebenenfalls das in dieser Weise erhaltene Produkt der Formel (I) in eines ihrer Salze oder Solvate umwandelt.

10. Verfahren zur Herstellung der Verbindungen nach Anspruch 1, worin A die Gruppe (a) darstellt und Y₁ und Y₂ ein Stickstoffatom bedeuten, **dadurch gekennzeichnet, daß** man eine Verbindung der Formel (V): 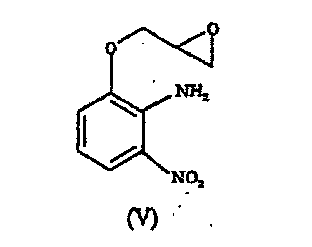 mit einer Verbindung der Formel (III), wie sie in Anspruch 8 definiert worden ist, umsetzt, die Nitrogruppe des in dieser Weise erhaltenen Produkts der Formel (VI): 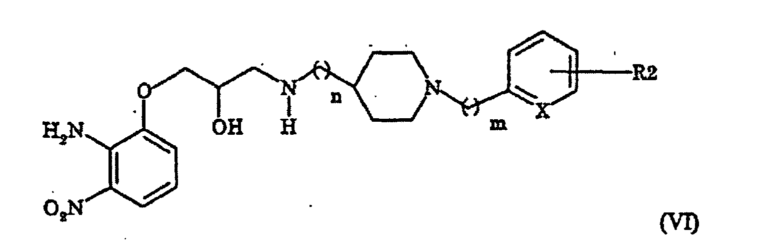 reduziert, das in dieser Weise erhaltene Produkt der Formel (VII): 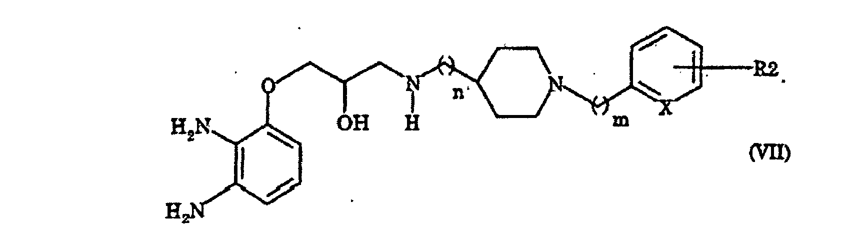 mit einem Carbonylierungsmittel umsetzt, das in dieser Weise erhaltene Produkt der Formel (I) isoliert und gegebenenfalls in eines seiner Salze oder Solvate umwandelt.

11. Verfahren nach Anspruch 10, **dadurch gekennzeichnet, daß** das Carbonylierungsmittel aus Carbonyldiimidazol und Phosgen ausgewählt ist.

12. Pharmazeutische Zubereitung enthaltend mindestens eine Verbindung nach Anspruch 1 als Wirkstoff.

13. Verwendung einer Verbindung nach Anspruch 1 für die Herstellung von Arzneimitteln, die für die Behandlung des Reizdarmsyndroms indiziert sind, oder eine modulierende Wirkung auf die Darmmotilität ausüben, eine lipolytische Wirkung, eine Anti-Fettsuchtwirkung, eine antidiabetische Wirkung, eine psychotrope Wirkung, eine antiglaukomatöse Wirkung, eine vernarbende Wirkung, eine antidepressive Wirkung, eine tocolytische Wirkung zur Vorbeugung oder Verzögerung von Frühgeburten, für die Behandlung und/oder die Prophylaxe des Dysmenorrhoe.

## Claims

1. Compound of formula (I): 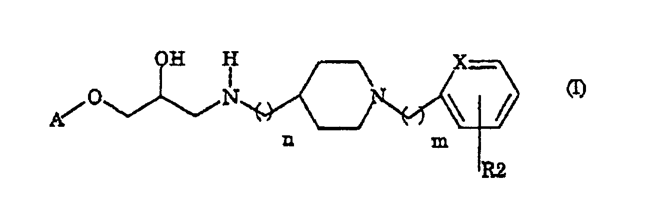 where
X is N or CH;
A represents a group of formula (a) or (b) 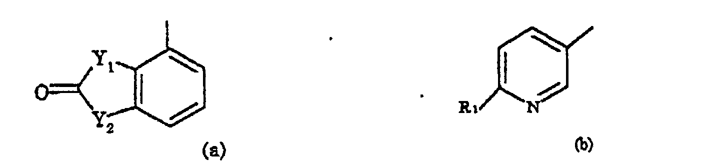
R₁ represents a hydrogen atom or an -NH₂, -NR₃R₄, -NR₃CO(C₁-C₄)Alk or -NR₃SO₂(C₁-C₄)Alk group;
R₂ represents a hydrogen or halogen atom or a (C₁-C₄)Alk, (C₁-C₄)alkoxy, -COOH, -COO(C₁-C₄)Alk, -CN, -CONR₃R₄, -NO₂, -SO₂NR₃R₄ or -NHSO₂(C₁-C₄)Alk group;
m and n each represent 0, 1 or 2;
R₃ and R₄ each represent a hydrogen atom or a (C₁-C₄)Alk group;
Y₁ and Y₂ each represent NH or O;
and their salts or solvates.

2. Compounds according to Claim 1, where X represents CH.

3. Compounds according to Claim 1, where X represents a nitrogen atom and the R₂ group is in the 5-position.

4. Compounds according to Claim 1, where the (C₁-C₄)Alk group is a methyl or ethyl group.

5. Compounds according to Claim 1, where R₂ is chosen from -COOH, -COO(C₁-C₄)Alk, -CN, -NO₂, -CONR₃R₄ and -NHSO₂-(C₁-C₄)Alk.

6. 3-[1-(5-Ethoxycarbonylpyrid-2-yl)-4-piperidinylamino]-1-(1,2-dihydro-2-oxobenzimidazol-4-yloxy)-2-propanol and its salts or solvates according to Claim 1.

7. 3-[1-(5-Ethoxycarbonylpyrid-2-yl)-4-piperidinylamino]-1-[2-aminopyrid-5-yloxy]-2-propanol and its salts or solvates according to Claim 1.

8. Process for the preparation of the compounds of Claim 1, **characterized in that** an epoxide of formula (II) : 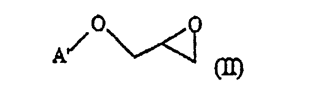 in which A' represents the group (a) or the group (b) in which R₁ is optionally protected, where (a), (b) and R₁ are as defined in Claim 1, is reacted with an amine of formula (III) 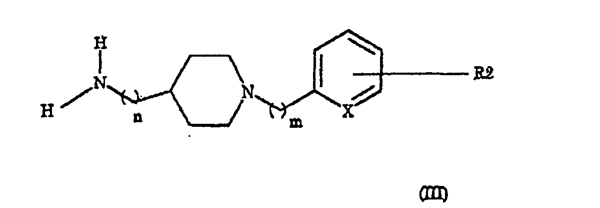 where m, n, R₂ and X are as indicated above, the protective group optionally present is removed and, optionally, the product of formula (I) thus obtained is converted into one of its salts or solvates.

9. Process for the preparation of a compound of Claim 1 where A represents a group (b) and R₁ is an NH₂ group, **characterized in that** a product of formula (II) as defined in Claim 8 where A' is the group (b) and R₁ is a group of formula: 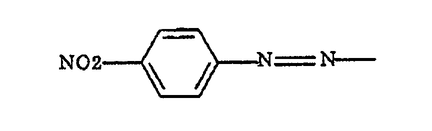 is reacted with an amine of formula III and the product of formula IV thus obtained: 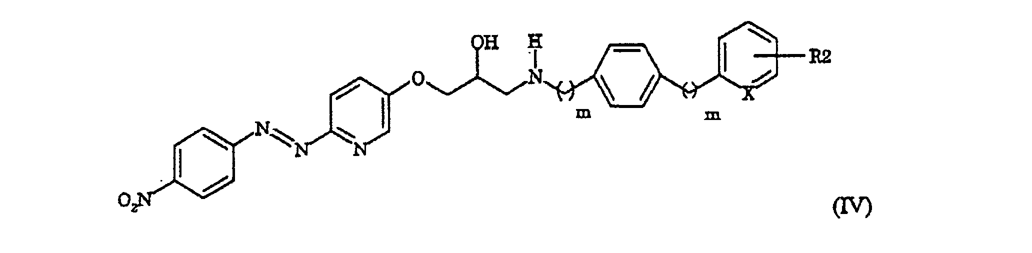 is subjected to a hydrogenation reaction in order to convert the 4-nitrophenyldiazenyl group to an amino group and, optionally, the product of formula (I) thus obtained is converted into one of its salts or solvates.

10. Process for the preparation of the compounds of Claim 1 where A represents the group (a) and Y₁ and Y₂ represent a nitrogen atom, **characterized in that** a compound of formula (V): 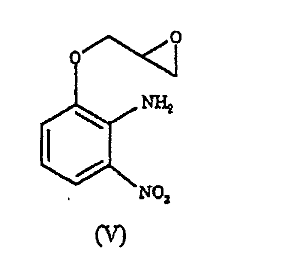 is reacted with a compound of formula (III) as defined in Claim 8, the nitro group of the product of formula (VI) thus obtained: 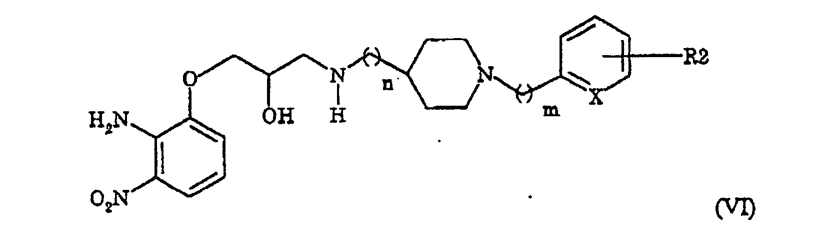 is reduced, the product of formula (VII) thus obtained: 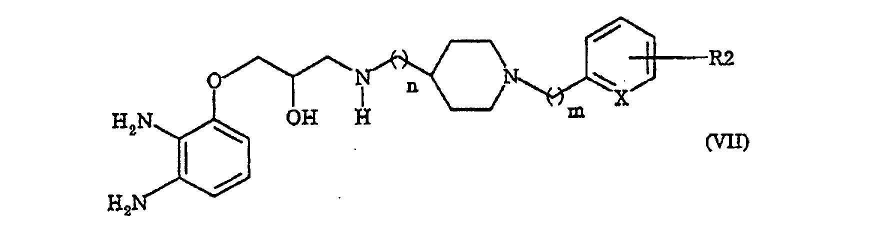 is treated with a carbonylation agent, the product of formula (I) thus obtained is isolated and, optionally, is converted into one of its salts or solvates.

11. Process according to Claim 10, **characterized in that** the carbonylation agent is chosen from carbonyldiimidazole and phosgene.

12. Pharmaceutical composition comprising at least one compound of Claim 1 as active principle.

13. Use of a compound according to Claim 1 for the preparation of medicaments indicated in irritable bowel syndrome, or with a modulating effect on intestinal motility, lipolytic agent, antiobesity agent, antidiabetic, psychotropic, antiglaucoma agent, cicatrizant or antidepressant, or as tocolytics for preventing or delaying premature labour or for the treatment and/or prophylaxis of dysmenorrhoea.
